# EUROPÄISCHE PATENTANMELDUNG

(11) **EP 2 446 846 A1**
(43) Veröffentlichungstag der Anmeldung: **02.05.2012**
(21) Anmeldenummer: 11187244.6
(22) Anmeldetag: 31.10.2011
(51) Int. Cl.: A61B 17/92, A61B 18/20

(54) **Anordnung zum Entfernen von Schrauben aus Knochenmaterial**

(30) Priorität: 29.10.2010 DE 102010060256
(71) Anmelder: CNC 2000 GmbH, 12277 Berlin (DE)
(72) Erfinder: Bakirci, Hawa, 12355 Berlin (DE)
(74) Vertreter: Weisse, Renate

(57) **Zusammenfassung**

Eine Anordnung zum in-vivo Extrahieren von Schrauben oder anderen Metallteilen (38) aus Knochen (36), ist dadurch gekennzeichnet, dass ein Betätigungsteil (30,32,26) fest mit dem Metallteil verbunden, insbesondere verschweißt oder verklebt wird. Die Schraube kann nach dem Verbinden mittels eines Motors herausgedreht werden.

## Beschreibung

### Technisches Gebiet

Die Erfindung betrifft eine Anordnung und ein Verfahren zum in-vivo Extrahieren von Metallteilen aus Knochen. Bei Frakturen und anderen Knochenschäden werden in der Medizin häufig Schrauben oder andere Metallteile eingesetzt um den beschädigten Knochenteil in seiner Lage zu stabilisieren oder Knochenteile zusammenzuführen. Wenn die Knochenteile wieder zusammengewachsen sind, wird die Schraube in einer zweiten Operation wieder entfernt. Dies dauert einige Wochen oder Monate. In dieser Zeit wächst Gewebe über dem Schraubenkopf.

Jede Operation erfolgt unter Narkose und ist mit gesundheitlichen Risiken verbunden. Es ist daher wünschenswert, die Operationsdauer so gering wie möglich zu halten.

Eine Schraube, die längere Zeit im Knochen eingeschraubt war, ist häufig schwer oder gar nicht zu entfernen. Dies kann vielfältige Ursachen haben. Der häufige Versuch die Schraube zu entfernen kann dazu führen, dass der Schraubensitz ausleiert. Dann kann die Schraube überhaupt nicht mehr mit einem Schraubendreher entfernt werden. Der Operateur verwendet dann einen Bohrer um die Schraube aus dem Knochen herauszubohren. Dabei können Späne in den Körper gelangen. Außerdem verlängert sich die Operationsdauer erheblich.

Die Verwendung magnetischer Verfahren zum Entfernen der Schraube ist bei Schrauben aus Titan nicht möglich. Auch Verfahren, die aus der Mechanik bekannt sind, etwa mit einer Zange oder anderen Werkzeugen können nicht eingesetzt werden, weil dabei ein Risiko weiterer Knochenbrüche besteht.

EP 1 447 054 A1 offenbart eine Vorrichtung zur Implantatentfernung. Ein Verankerungselement in Form einer Schraube wird in das Implantat eingeschraubt bevor dieses im Knochen eingesetzt wird. An dem Verankerungselement ist ein Koppelelement in Form eines Seils mit einer Schlaufe befestigt, beispielsweise verschweißt. Die Schlaufe wird unter der Haut abgelegt und ermöglicht so das Wiederauffinden des Implantats.

### Offenbarung der Erfindung

Es ist Aufgabe der Erfindung, eine Vorrichtung der eingangs genannten Art zu schaffen, mit welchem auch defekte Schrauben oder andere Metallteile ohne zusätzliches Gesundheitsrisiko aus einem Knochen entfernt werden können. Erfindungsgemäß wird die Aufgabe dadurch gelöst, dass ein Betätigungsteil fest mit dem Metallteil verbunden, insbesondere verschweißt oder verklebt wird. Wenn das Metallteil eine Schraube ist, kann das mit der Schraube verbundene Betätigungsteil nach dem Verbinden leicht und schnell herausgedreht werden. Hierzu kann insbesondere ein Motor vorgesehen sein, der mit dem Betätigungsteil verbunden ist. Vorzugsweise wird das Betätigungsteil mittels Laserschweißen mit dem Metallteil verschweißt.

Das erfindungsgemäße Verfahren ermöglicht die Entfernung ausgeleierter oder festsitzender Schrauben aus dem Knochen während einer Operation ohne dass die Operation sich wesentlich verlängert. Das Betätigungsteil wird mit der Schraube verbunden und beide Teile werden gemeinsam herausgedreht. Der Knochen wird dabei geschont.

Zur Verwirklichung des erfindungsgemäßen Verfahrens ist eine Anordnung zum in-vivo Extrahieren von Metallteilen aus Knochen mit einem Betätigungsteil vorgesehen, die, gekennzeichnet ist durch Mittel zum Verschweißen oder Verkleben des Betätigungsteils mit dem Metallteil.

Das Betätigungsteil kann ein metallisches Ende aufweisen und es kann ein Laser vorgesehen sein, mit dem eine Schweißverbindung zwischen dem metallischen Ende des Betätigungsteils und dem zu extrahierenden Metallteil herstellbar ist.

Bei einer bevorzugten Ausgestaltung der Erfindung weist das Betätigungsteil eine Bohrung auf, durch welche der Laserstrahl des Lasers zum Verbindungsbereich geführt ist. Die Bohrung mündet quasi am knochen-seitigen Ende des Betätigungsteils. Dieses wird in die Schraube gesteckt, soweit das möglich ist. Anschließend wird der Laser eingekoppelt und die Schraube an das Betätigungsteil geschweißt. Die Schraube kann dann leicht herausgedreht werden.

In einer weiteren Ausgestaltung der Erfindung hat das Betätigungsteil die äußere Form eines Schraubendrehers hat und die Bohrung ist koaxial bis in die Spitze geführt.

In einer besonders bevorzugten Ausgestaltung der Erfindung weist das Betätigungsteil ein auswechselbares Endteil auf. Das Endteil kann mittels einer Kupplung an dem Griff oder Motor angeschlossen sein. Die Verwendung eines auswechselbaren Endteils ermöglicht es dieses als Einmal-Artikel auszuführen. Es wird bei jedem Patienten ein neues Endteil verwendet. Dadurch können erforderliche Hygienestandards erreicht werden. Statt eines Motors kann auch ein handbetätigtes Betätigungsteil eingesetzt werden.

In einer weiteren Ausgestaltung der Erfindung ist ein Steuergerät vorgesehen, in welchem eine Laserlichtquelle angeordnet ist, dessen Licht zusammen mit eventuell vorhandenen Stromkabeln in einem Schlauch zum Betätigungsteil geführt ist. Der Laserstrahl kann durch einen Lichtleiter geleitet werden. In dem Schlauch können Stromkabel zur Energieversorgung des Motors und Steuerleitungen angeordnet sein. Das Kabelpaket ist durch den Schlauch vor Verunreinigungen und äußeren Einflüssen geschützt. Er ermöglicht eine flexible Handhabung des Betätigungsteils während der Operation.

Ausgestaltungen der Erfindung sind Gegenstand der Unteransprüche. Ein Ausführungsbeispiel ist nachstehend unter Bezugnahme auf die beigefügten Zeichnungen näher erläutert.

### Kurze Beschreibung der Zeichnungen

- Fig. 1: ist eine schematische Darstellung einer Anordnung zum in-vivo Extrahieren von Metallteilen aus Knochen.
- Fig.2: ist ein Querschnitt durch das Betätigungsteil einer Anordnung aus Fig.1 und den Knochen vor der Extraktion.
- Fig.3: illustriert die Spitze des Endteils eines Betätigungsteils aus Fig.2 während des Schweißprozesses.

### Beschreibung des Ausführungsbeispiels

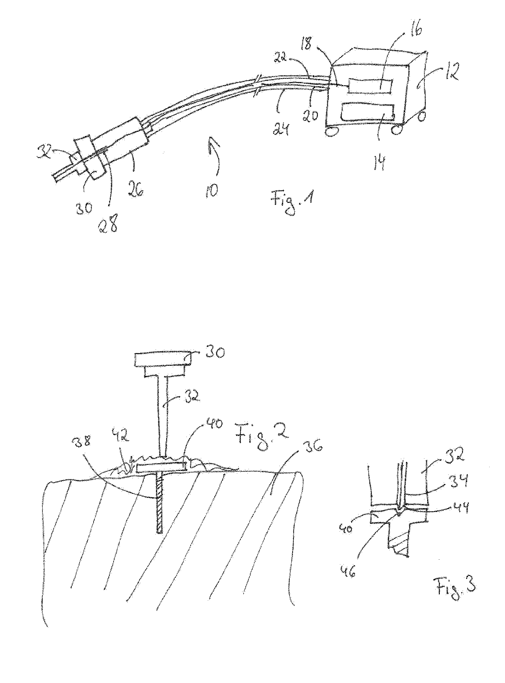

Figur 1 zeigt eine Anordnung zum in-vivo Extrahieren von Schrauben oder vergleichbaren Metallteilen, die allgemein mit 10 bezeichnet ist. Die Anordnung 10 umfasst ein transportables oder fahrbares Steuergerät 12 mit einem Display 14, einer Stromversorgung (nicht dargestellt) und einem Laser 16. Die von dem Laser 16 erzeugte Laserstrahlung wird auf herkömmliche Weise in einen Lichtleiter 18 eingekoppelt.

Der Lichtleiter 18 ist zusammen mit einem Steuerkabel 20 und einem Stromkabel 22 in einem flexiblen Schlauch 24 geführt. Der Schlauch 24 hält diese Versorgungskabel 18, 20 und 22 zusammen und schützt diese vor Umwelteinflüssen. Der Schlauch 24 mit dem Kabelpaket mündet an den Anschlüssen eines Handmotors 26, der als Elektromotor ausgebildet ist. Der Handmotor 26 treibt eine Welle 28 an. Die Stromversorgung des Handmotors 26 erfolgt über das Stromkabel 22. Die Steuerung des Handmotors erfolgt im vorliegenden Ausführungsbeispiel über das Steuerkabel 20, über welches Steuersignale von dem Steuergerät 12 an den Motor übertragen werden. In einem nicht dargestellten Ausführungsbeispiel erfolgt die Steuerung des Motors direkt am Motor 26. Die von dem Motor 26 angetriebene Welle ist über eine Kupplung 30 mit einem Endteil 32 verbunden. Das Endteil 32 hat im vorderen Bereich die äußere Form eines Schraubendrehers, dessen Größe an die Abmessungen der eingesetzten Schrauben angepasst ist.

Der Lichtleiter 18 ist durch die Welle 28 geführt und endet in einer Längsbohrung 34 (Fig.3) im Endteil 32. An dem der Kupplung abgewandten, leicht zugespitzten Ende des Endteils tritt die Laserstrahlung aus dem Lichtleiter 18 aus.

Die beschriebene Anordnung 10 arbeitet wie folgt:
In dem Knochen 36 - dargestellt in Figur 2 - befindet sich eine zu extrahierende Schraube 38 mit Gewinde. Die Schraube 38 hat einen Kopf 40. Während des Heilungsprozesses kann der Kopf 40 der Schraube mit Gewebe 42 überwachsen und zuwuchern. Das Endteil 32 wird mit dem zugespitzten Ende 44 in die passende Nut 46 gesteckt, wie dies in Figur 3 schematisch dargestellt ist. Anschließend wird der Laser 16 aktiviert. Die Laserstrahlung ist so stark, dass der Schraubenkopf 40 mit dem Endteil 44 fest verschweißt wird.

Anschließend wird der Motor 26 eingeschaltet. Die Rotationsbewegung der Welle 28 wird auf das Endteil 32 und die damit verschweißte Schraube 38 übertragen. Die Drehgeschwindigkeit des Motors 26 ist so eingestellt, dass die festgewachsene Schraube 38 vorsichtig und ohne weitere Verletzungen herausgedreht wird. Die Entfernung der Schraube erfolgt dabei so schnell, dass die Operationsdauer sich nicht wesentlich verlängert. Dadurch wird der Patient geschont.

Nach Entfernen der festgeschweißten Schraube wird die Kupplung 30 gelöst. Das Endteil 32 kann zusammen mit der Schraube 38 entsorgt werden. Für die nächste Operation wird ein neues Endteil 32 mit der Kupplung 30 am Motor 26 befestigt.

## Patentansprüche

1. Verfahren zum in-vivo Extrahieren von Metallteilen (38) aus Knochen (36), **dadurch gekennzeichnet, dass** ein Betätigungsteil (30, 32, 26) unmittelbar vor der Extraktion fest mit dem Metallteil verbunden, insbesondere verschweißt oder verklebt wird.

2. Verfahren nach Anspruch 1, **dadurch gekennzeichnet, dass** das Metallteil eine Schraube (38) ist und das mit der Schraube verbundene Betätigungsteil (26, 30, 32) nach dem Verbinden zusammen mit der Schraube herausgedreht wird.

3. Verfahren nach Anspruch 1 oder 2, **dadurch gekennzeichnet, dass** das Betätigungsteil (32) mittels Laserschweißen mit dem Metallteil verschweißt wird.

4. Anordnung zum in-vivo Extrahieren von Metallteilen aus Knochen mit einem Betätigungsteil (32), **gekennzeichnet durch** Mittel (16) zum Verschweißen oder Verkleben des Betätigungsteils (26, 30, 32) mit dem Metallteil (38).

5. Anordnung nach Anspruch 4, **dadurch gekennzeichnet, dass** das Betätigungsteil (26, 30, 32) ein metallisches Endteil (32) aufweist und ein Laser (16) vorgesehen ist, mit dem eine Schweißverbindung zwischen dem metallischen Endteil des Betätigungsteils (26, 30, 32) und dem zu extrahierenden Metallteil (38) herstellbar ist.

6. Anordnung nach Anspruch 5, **dadurch gekennzeichnet, dass** das Betätigungsteil eine Bohrung (34) aufweist, durch welche der Laserstrahl des Lasers (16) zum Verbindungsbereich geführt ist.

7. Anordnung nach Anspruch 6, **dadurch gekennzeichnet, dass** das Betätigungsteil (32) die äußere Form eines Schraubendrehers hat und die Bohrung (34) koaxial bis in die Spitze (44) geführt ist.

8. Anordnung nach einem der Ansprüche 4 bis 7, **dadurch gekennzeichnet, dass** das Betätigungsteil ein auswechselbares Endteil (32) aufweist.

9. Anordnung nach Anspruch 8, **dadurch gekennzeichnet, dass** das auswechselbare Endteil (32) mittels einer Kupplung (30) an einen Motor (26) angeschlossen ist.

10. Anordnung nach einem der Ansprüche 5 bis 9, **dadurch gekennezeichnet, dass** ein Steuergerät (12) vorgesehen ist, in welchem eine Laserlichtquelle (16) angeordnet ist, dessen Licht zusammen mit eventuell vorhandenen Stromkabeln (20) in einem Schlauch (24) zum Betätigungsteil (26, 30, 32) geführt ist.
